# EUROPEAN PATENT APPLICATION

(11) **EP 4 428 237 A1**
(43) Date of publication of application: **11.09.2024**
(21) Application number: 22889245.1
(22) Date of filing: 01.11.2022
(51) Int. Cl.: C12N 15/113, C12N 15/67, C12N 15/85, C12N 5/10, A61K 48/00, A61P 1/16

(54) **ISOLATED NUCLEIC ACID MOLECULE AND USE THEREOF**

(30) Priority: 02.11.2021 CN 202111289091
(71) Applicant: Gritgen Therapeutics Limited, Suzhou, Jiangsu 215123 (CN)
(72) Inventor: YU, Tenghui, Suzhou, Jiangsu 215123 (CN); YANG, Zhou, Suzhou, Jiangsu 215123 (CN); ZHANG, Jianghong, Suzhou, Jiangsu 215123 (CN); SHEN, Lijun, Suzhou, Jiangsu 215123 (CN); RAO, Yi, Suzhou, Jiangsu 215123 (CN)
(74) Representative: Weickmann & Weickmann PartmbB
(86) International application number: PCT/CN2022/128833
(87) International publication number: WO 2023/078220

(57) **Abstract**

An isolated nucleic acid molecule and the use thereof. The nucleic acid molecule comprises a liver-specific expression regulatory element and a target gene, and the liver-specific expression regulatory element can increase the expression amount and/or activity of the target gene. Further provided are a vector a diagnostic or pharmaceutical composition containing the isolated nucleic acid molecule, and the use thereof.

## Description

### Technical Field

The application relates to the field of biomedicine and, in particular, to an isolated nucleic acid molecule and use thereof.

### BACKGROUND OF THE INVENTION

With the development of molecular biology, gene therapy has been used in experiments and clinical applications for treating a series of disorders and diseases, comprising related diseases caused by protein synthesis dysfunction in the liver. Due to its protein post-translational modification function, the liver has become an important target organ for genetic treatment of hereditary blood diseases. Liver gene therapy can introduce a functional gene into an individual with gene defects, and allow the gene to express efficiently in vivo for a long time. However, at present, the main factor restricting the liver gene therapy is the low-level expression of gene in the liver. One of the reasons for the low-level expression lies in weak transcriptional activity. To enhance the high-level expression of gene in vivo, it is necessary to construct a high-efficiency expression regulatory sequence in the liver.

In order to promote the expression of a gene of interest in a host cell, the gene of interest is usually comprised in a construct that also contains various regulatory elements necessary for expressing the gene of interest. These regulatory elements may comprise, for example, a promoter, an enhancer, an initiation signal, a termination signal, and other regulating elements. The ideal synergy between elements can not only promote the stable expression of the gene of interest in the host cell, but also achieve an expression level that is sufficient to realize the function or activity of the gene of interest. The promoter and other regulatory elements determine cell type specificity, transduction efficiency, expression level, and duration. It may be difficult to achieve the high-efficiency stable expression of the gene of interest in the host cell. Therefore, there is an urgent need of developing a nucleic acid molecule or a vector to facilitate the high-efficiency stable expression of a gene of interest in a host cell.

### SUMMARY OF THE INVENTION

The application provides an isolated nucleic acid molecule, which may comprise a liver-specific expression regulatory element, a gene of interest (e.g., a polynucleotide encoding a B domain-deleted FVIII variant), and a polyadenylation signal sequence (e.g., Ks13 poly A), and the gene of interest in the isolated nucleic acid molecule as defined has a high expression level and/or high activity. Compared with an expression regulatory element with a nucleotide sequence as set forth in SEQ ID NO: 10, the liver-specific expression regulatory element of the application can increase the expression level and/or activity of said gene of interest. Compared with a polyadenylation signal sequence with a nucleotide sequence as set forth in SEQ ID NO: 8, the polyadenylation signal sequence of the application can increase the expression level and/or activity of said gene of interest.

In one aspect, the application provides an isolated nucleic acid molecule, comprising, from 5' to 3 ', a liver-specific expression regulatory element and a gene of interest operably linked to said liver-specific expression regulatory element, wherein compared with an expression regulatory element with a nucleotide sequence as set forth in SEQ ID NO: 10, said liver-specific expression regulatory element increases the expression level and/or activity of said gene of interest by at least 50%.

In some embodiments, said liver-specific expression regulatory element comprises: a) a promoter derived from a dog serpinA1 gene or a functional fragment thereof; and b) a promoter derived from a Xenopus laevis albumin gene or a functional fragment thereof.

In some embodiments, said liver-specific expression regulatory element comprises, in order from 5' to 3': said promoter derived from a dog serpinA1 gene or a functional fragment thereof; and said promoter derived from a Xenopus laevis albumin gene or a functional fragment thereof.

In some embodiments, said liver-specific expression regulatory element comprises a nucleotide sequence as set forth in SEQ ID NO: 1.

In some embodiments, the isolated nucleic acid molecule as defined comprises a polyadenylation signal sequence located at a 3 '-terminal of said gene of interest.

Compared with an polyadenylation signal sequence in the prior art, said polyadenylation signal can increase the expression level and/or activity of said gene of interest by at least 20% (for example, at least 25%, at least 30%, at least 35%, at least 40%, at least 45%, at least 50%, at least 55%, at least 60%, at least 65%, at least 70%, at least 75%, at least 80%, at least 90%, at least 99% or more). Compared with a BGH poly A and/ an SV40 poly A, said polyadenylation signal can increase the expression level and/or activity of said gene of interest by at least 20%.

In some embodiments, said polyadenylation signal is a polyadenylation signal derived from Common vole polyomavirus.

In some embodiments, said polyadenylation signal is a polyadenylation signal derived from Common vole polyomavirus isolate KS13.

In some embodiments, said polyadenylation signal comprises a nucleotide sequence as set forth in SEQ ID NO: 3.

In some embodiments, said gene of interest encodes a protein of interest, and said protein of interest comprises a reporter protein, a therapeutic protein and/or a prophylactic protein.

In some embodiments, said gene of interest encodes a B domain-deleted Factor VIII (FVIII) variant. In some embodiments, said gene of interest encodes a GFP. In some embodiments, said gene of interest encodes luciferase.

In some embodiments, said FVIII variant comprises an amino acid sequence as set forth in SEQ ID NO: 7.

In some embodiments, said gene of interest comprises a nucleotide sequence as set forth in SEQ ID NO: 2 or 6.

In some embodiments, the isolated nucleic acid molecule as defined comprises, in order from 5' to 3': said liver-specific expression regulatory element, said gene of interest, and said polyadenylation signal sequence.

In some embodiments, the isolated nucleic acid molecule as defined further comprises AAV inverted terminal repeats ITRs located at a 5'-terminal of the liver-specific expression regulatory element and at a 3'-terminal of said polyadenylation signal sequence.

In some embodiments, said AAV ITR is derived from an AAV serotype selected from the group consisting of: AAV5 and AAV2.

In some embodiments, said AAV ITR comprises a nucleotide sequence as set forth in any one of SEQ ID NOs: 4-5.

In some embodiments, the isolated nucleic acid molecule as defined comprises a nucleotide sequence as set forth in SEQ ID NO: 9.

In another aspect, the application provides a vector, comprising the isolated nucleic acid molecule, as defined.

In some embodiments, the vector as defined is a viral vector or a polynucleotide vector.

In some embodiments, the vector as defined is a plasmid, a cosmid or a transposon.

In some embodiments, the vector as defined is a viral vector and said viral vector comprises an AAV vector.

In some embodiments, said AAV vector is an AAV8 vector.

In another aspect, the application provides a host cell, comprising the isolated nucleic acid molecule as defined and/or the vector as defined.

In another aspect, the application provides a diagnostic or pharmaceutical composition, comprising the isolated nucleic acid molecule as defined, the vector as defined, and/or the host cell as defined.

In another aspect, the application provides a method of expressing a gene of interest, comprising introducing the isolated nucleic acid molecule as defined or the vector as defined into a host cell, and allowing said gene of interest to be expressed in said host cell.

In another aspect, the application provides a kit, comprising the isolated nucleic acid molecule as defined, the vector as defined, and/or the host cell as defined.

In another aspect, the application provides a method of delivering the isolated nucleic acid molecule as defined into a mammal or a mammalian cell, comprising administering the isolated nucleic acid molecule as defined or the vector as defined to said mammal or mammalian cell, or contacting said mammal or mammalian cell with the isolated nucleic acid molecule as defined or the vector as defined.

In another aspect, the application provides a use of the isolated nucleic acid molecule as defined, the vector as defined, and/or the host cell as defined in the preparation of a medicament for treating, alleviating, and/or preventing an FVIII-related disease or disorder.

In some embodiments, the disease or disorder comprises hemophilia A, thrombocytopenia, and/or coagulopathy.

In another aspect, the application provides a method of treating, alleviating and/or preventing an FVIII-related disease or disorder, comprising administering to a subject in need thereof the isolated nucleic acid molecule as defined, the vector as defined, and/or the host cell as defined.

In some embodiments, the disease or disorder comprises hemophilia A, thrombocytopenia, and/or coagulopathy.

In another aspect, the application provides a use of the isolated nucleic acid molecule as defined, the vector as defined, and/or the host cell as defined for treating, alleviating, and/or preventing an FVIII-related disease or disorder.

In some embodiments, the disease or disorder comprises hemophilia A, thrombocytopenia, and/or coagulopathy.

Other aspects and advantages of the application can be readily perceived by those skilled in the art from the detailed description below. The detailed description below only shows and describes the exemplary embodiments of the application. As would be appreciated by those skilled in the art, the content of the application allows those killed in the art to change the specific embodiments disclosed without departing from the spirit and scope involved in the application. Accordingly, the accompanying drawings and the description of the application are merely for an exemplary but not restrictive purpose.

### BRIEF DESCRIPTION OF THE DRAWINGS

The specific features of the application involved in the application are listed in the appended claims. The features and advantages of the application involved in the application can be better understood with reference to the exemplary embodiments and the accompanying drawings described in detail below. The drawings are described briefly as follows.

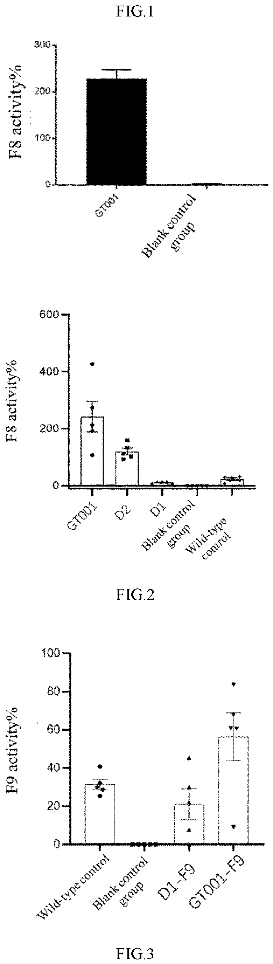

FIG. 1 shows that the isolated nucleic acid molecule of the application can restore FVIII expression in mice with hemophilia A.
FIG. 2 shows the expression activity of the gene of interest FVIII over different regulatory elements. GT001 represents a specific expression vector group of the application; D2 represents an expression vector group after polyA replacement; D1 represents an expression vector group after promoter replacement; blank control represents FVIII knockout group; wild-type control represents wild mouse group.
FIG. 3 shows the expression activity of the expression nucleic acid molecule of the application for optionally expressing a gene of interest. Wild-type control represents wild mouse group; blank control represents FVIII knockout group; D1-F9 represents expression vector group after promoter replacement used to express FIX; GT001-F9 represents specific expression vector group of the application used to express FIX.

### DETAILED DESCRIPTION

The embodiments of the invention of the application will be illustrated by specific examples below. Those familiar with this technology can easily understand other advantages and effects of the invention of the application from the content disclosed herein.

In the application, the term "isolated" generally refers to that a biological component (such as a nucleic acid molecule, a protein, a virus, or a cell) is purified to be substantially free of other cellular components (such as other chromosomes and extrachromosomal DNA and RNA, proteins and cells) related thereto in a naturally occurring condition. The isolated nucleic acid molecule and protein comprise those purified by a standard purification method. This term also involves nucleic acid molecules and proteins prepared by recombinant expression in host cells, and chemically synthesized nucleic acid molecules and proteins.

In the application, the term "dog serpinAl gene" generally refers to a gene encoding α 1-antitrypsin. In some cases, the dog as described may be a dog with the Latin name Canis lupus familiaris. In another case, the "dog serpinAl gene" may comprise a nucleotide sequence as set forth from a position 63,388,498 to a position 63,400,377 of a chromosome 8 (for its sequence, refer to NCBI Accession No. NC_006590.3), or a variant thereof. The promoter derived from the "dog serpinAl gene" may comprise the nucleotide sequence as set forth from a position 63,398,647 to a position 63,398,492 of a downstream strand of the chromosome 8, or a variant thereof.

In the application, the term "Xenopus laevis albumin gene" generally refers to a nucleic acid sequence comprising that as set forth in GenBank Accession No. Z26825.1. Its promoter may comprise a nucleotide sequence from position 1540 to position 1605 of the above-mentioned sequence.

In the application, the term "codon" generally refers to an oligonucleotide consisting of three nucleotides encoding a defined amino acid. Due to the degeneracy of the genetic code, some amino acids are encoded by more than one codon. These different codons encoding the same amino acid have different relative frequencies of use in individual host cells. Therefore, a specific amino acid may be encoded by a set of different codons. Similarly, the amino acid sequence of a polypeptide may be encoded by different nucleic acids. Therefore, a specific amino acid may be encoded by a set of different codons, where each of these codons has a given frequency of use in a host cell. In the application, the term "codon-optimized" means replacing one, at least one, or more than one codon in a polypeptide-encoding nucleic acid with different codons having different frequencies of use in a corresponding cell. In the application, the term "codon-optimized polynucleotide" refers to a polypeptide-encoding nucleic acid modified to have improved expression in a cell, that is obtained by replacing one, at least one, or more than one codon in a parent polypeptide-encoding nucleic acid with codons having different relative frequencies of use in the cell and encoding the same amino acid residue.

In the application, the term "FVIII" or "Factor FVIII" generally refers to a coagulation factor VIII. The FVIII is a protein required for effective blood coagulation and can function as a cofactor in coagulation. The FVIII concentration of approximately 100 ng/ml in blood is considered to be within a normal range. Deficiency of FVIII is associated with hemophilia A, which causes severe disease when a subject has less than about 1 ng of FVIII per milliliter of blood.

In the application, the term "FVIII variant" generally refers to a protein that has undergone certain modifications on the basis of wild-type FVIII. This modification may include deletion of its B domain. That is, the so-called FVIII variant refers to a B domain-deleted FVIII protein.

In the application, the term "promoter" generally refers to an essential nucleic acid sequence used to promote the transcription of a downstream (3'-terminal) or upstream (5'-terminal) nucleic acid sequence. A promoter is generally located in the vicinity of a gene to be transcribed thereby. The promoter also comprises a terminal enhancer or repressor element, which can be located several thousands of base pairs from a transcription start site.

In the application, the term "functional fragment" is a fragment of a polypeptide or polynucleotide that retains the same activity or ability as its larger counterpart (e.g., a promoter). The activity level of the functional fragment may be the same as, or lower or higher than, the activity level of the mentioned larger counterpart. For example, the functional fragment of a promoter may be composed of polynucleotides fewer than those of the promoter, but still retain the ability of promoting transcription.

In the application, the term "gene of interest" generally refers to an exogenous DNA or cDNA, which is comprised in a nucleic acid molecule, vector, host cell or kit and is capable of encoding a gene product. The gene product may be a polypeptide, a protein, or a polynucleotide (e.g., antisense DNA, miRNA, siRNA, and shRNA) that is inherently functional or active.

In the application, the term "reporter protein" generally refers to a protein that provides an analytically identifiable signal by biochemical nature, thus permitting detection. Commonly used reporter proteins may be enzymes, fluorophores, and chemiluminescent or electrochemiluminescent proteins.

In the application, the term "therapeutic protein" generally refers to a protein that can be used to treat, prevent, or ameliorate a disease, condition, or disorder.

In the application, the term "prophylactic protein" generally refers to a protein that can be used to prevent a condition or disease before any symptoms of the condition or disease are detected.

In the application, the term "operably linked" generally refers to that a first nucleic acid sequence and a second nucleic acid sequence are operably linked when the first and second nucleic acid sequences are in a functional relationship. For example, if a promoter affects the transcription or expression of a coded sequence, the promoter and the coded sequence are operably linked.

In the application, the terms "polyadenylation signal sequence" and "polyA signal sequence" generally refer to a nucleic acid sequence for inducing the cleavage and polyadenylation of a primary transcript of a specific nucleic acid sequence segment. The polyadenylation signal sequence may be selected from polyadenylation signal sequences derived from SV40, a bovine growth hormone (BGH) gene, an immunoglobulin gene, and a thymidine kinase gene (tk, e.g., a herpes simplex virus thymidine kinase polyadenylation signal).

In the application, the term "vector" generally refers to a nucleic acid molecule capable of self-replication in a suitable host cell, which transfers the inserted nucleic acid molecule into a host cell and/or between host cells. The vector may involve vectors primarily used for insertion of DNA or RNA into cells, vectors primarily used for replication of DNA or RNA, and vectors primarily used for expression of transcription and/or translation of DNA or RNA. The vector may also involve vectors having a variety of the above-mentioned functions. The vector may be a polynucleotide capable of being transcribed and translated into a polypeptide when introduced into a suitable host cell. Typically, the vector can produce a desired expression product by culturing a suitable host cell containing the vector.

In the application, the term "AAV vector" or "virus vector" generally refers to a vector derived from naturally occurring and available adeno-associated virus and artificial AAV. The AAV may comprise different serotypes such as AAV1, AAV2, AAV3, AAV4, AAV5, AAV6, AAV7, AAV8, AAV9, AAV10, AAV11, AAV12 or AAV13, as well as any AAV variants or a mixture thereof. Inverted terminal repeats (ITR) are generally present at both ends of an AAV genome. The term "ITR" or "inverted terminal repeat" refers to a nucleic acid sequence segment that is present in an AAV and/or a recombinant AAV. It can form a T-shaped palindrome structure required for completing AAV solubilization and a latent life cycle.

In the application, the term "host cell" generally refers to a cell, such as a mammalian cell, that has been transformed with a nucleic acid sequence or is capable of being transformed with a nucleic acid sequence to consequently express a gene of interest. The "host cell" comprises a parent cell and a progeny cell thereof, and it is enough as long as the progeny cell can be transferred into a gene of interest or the gene of interest already exists, regardless of whether the progeny cell is identical to the original parent cell in terms of morphology or genetic structure.

Therefore, the application comprises the variants of gene and protein (e.g., the variant of a protein-encoding polynucleotide described herein), which retain one or more biological activities. Such variant of protein or polypeptide comprises a protein or polypeptide that has been or can be modified using a recombinant DNA technology in such a way that the protein or polypeptide has altered or additional properties. For example, the variant endows the protein with an enhanced stability in plasma or with an enhanced activity. The variant may be different from a reference sequence, such as a naturally occurring polynucleotide, protein or peptide. At a nucleotide sequence level, the naturally occurring variant gene and the non-naturally occurring variant gene would show typically at least about 50%, more typically at least about 70%, and even more typically at least about 80% (90% or higher), identity with a reference gene. At an amino acid sequence level, a naturally-occurring mutant protein and a non-naturally occurring mutant protein would show typically at least about 70%, more typically at least about 80%, and even more typically at least about 90% or higher, identity with a reference protein, but a largely non-identity region is allowed in a non-conserved region (for example, the proportion of identical portions is less than 70%, such as less than 60%, less than 50%, or even less than 40%). In other embodiments, the sequence has at least 60%, 70%, 75% or higher identity (e.g., 80%, 85%, 90%, 95%, 96%, 97%, 98%, 99% or higher identity) with a reference sequence. For those skilled in the art, procedures for introducing the changes of nucleotide and amino acid into a polynucleotide, protein, or peptide are known (see, e.g., Sambrook et al. (1989)).

In the application, the terms "sequence similarity", "identity", "homology" and their grammatical variants generally refer to the fact that two or more entities are considered to be the same if they are "aligned" sequences. Therefore, for example, when two polypeptide sequences are the same, they have the same amino acid sequence at least within a region or portion to which a reference is made. If two polynucleotide sequences are the same, they have the same polynucleotide sequence at least within a region or portion to which a reference is made. The identity may involve a defined zone (region or domain) of the sequence. A "zone" or "region" of identity refers to the same portion between two or more entities to which a reference is made. Therefore, if two protein or nucleic acid sequences are the same in one or more sequence zones or regions, they show identity in that region. "Aligned" sequences refer to a plurality of polynucleotide or protein (amino acid) sequences, which, compared with a reference sequence, often contain a base or an amino acid (gap) that is supplemented for deletion or added. The degree of identity (homology) between two sequences can be determined using a computer program and a mathematical algorithm. Such an algorithm for calculating percentage sequence identity (homology) is generally used to calculate a sequence gap and mismatch between compared regions or zones. For example, BLAST (e.g., BLAST 2.0) search algorithm (see, e.g., Altschul et al., J. Mol. Biol. 215: 403 (1990), publicly available through NCBI) has the following exemplary search parameters: mismatch: -2, gap opening: 5, and gap extension: 2.

In the application, the term "kit" generally refers to a combination of reagents and other materials. Kits are contemplated to contain reagents such as buffers, protein stabilizing reagents, signal generating systems (e.g., fluorescent signal generating systems), antibodies, control proteins, and test vessels (e.g., microtiter plates, etc.). The term "kit" is not intended to be limited to a specific combination of reagents and/or other materials. In an embodiment, the kit also contains instructions for using the reagents. A test kit may be packaged in any suitable manner. Typically, it has ingredients in a single container or, if necessary, in multiple containers and an instruction sheet for conducting the test. In some embodiments, the kit also preferably includes a positive control sample. The kit may be prepared in a variety of ways known in the art.

In the application, the term "administration" generally refers to the delivery of a substance (e.g., the isolated nucleic acid molecule or vector described herein) to a human or animal in need thereof by any route known in the art. Pharmaceutical carriers and formulations or compositions are also well known in the art. Routes of administration may include: intravenous, intramuscular, intradermal, subcutaneous, transdermal, mucosal, intratumoral or mucosal. Alternatively, these terms may refer to the delivery of the isolated nucleic acid molecule or vector as described herein to cells or cultured cells and/or the cells or organs of a subject. Such administration or introduction may occur in vivo, in vitro, or in vivo first and then in vitro. The isolated nucleic acid molecule or vector described herein can be introduced into a cell by: transfection, which generally refers to, by physical means (e.g., calcium phosphate transfection, electroporation, microinjection, or lipofection), the insertion of heterologous DNA into the cell; infection, which generally refers to the introduction of an infectious agent (i.e., a virus); or transduction, which generally refers to the stable infection of the cell by a virus, or the transfer of genetic material from one microorganism to another by a viral agent (e.g., a bacteriophage).

In the application, the term "contacting" generally refers to the introduction of a substance (e.g., the isolated nucleic acid molecule or vector described herein) into a subject (e.g., a mammal or a mammal cell) by any route known in the art and bringing the substance into contact with cells in vivo. The contacting may be direct or indirect. For example, it may be direct injection of cells by microinjection. For another example, the substance can be brought into contact with the cells in vivo by being provided in a culture medium surrounding the cells or administered to a subject.

In the application, the term "hemophilia" generally refers to a blood coagulation disorder. "Hemophilia A" generally refers to a recessive X-linked gene disorder in individuals lacking functional factor VIII.

In the application, the term "comprise" generally refers to the inclusion of explicitly specified features, but not excluding other elements.

In the application, the term "about" generally refers to a variation within a range of 0.5%-10% above or below a specified value, for example, a variation within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, and 10% above or below a specified value.

### Isolated Nucleic Acid Molecule

### Liver-specific expression regulatory element

In one aspect, the application provides an isolated nucleic acid molecule, which may comprise a liver-specific expression regulatory element and a gene of interest operably linked to said liver-specific expression regulatory element, wherein compared with an expression regulatory element with a nucleotide sequence as set forth in SEQ ID NO: 10, the liver-specific expression regulatory element may increase the expression level and/or activity of the gene of interest by at least 50%. For example, the liver-specific expression regulatory element may increase the expression level and/or activity of the gene of interest by at least 50%, at least 55%, at least 60%, at least 65%, at least 70%, at least 75%, at least 80%, at least 85%, at least 90%, at least 95%, or at least 99%. The expression level here may be determined in the liver or hepatocytes, or may also be determined in the blood.

In the application, the term "expression regulatory element" generally refers to a nucleic acid sequence, such as an enhancer and a promoter, which affects the expression of an operably linked polynucleotide. These elements may be operated in cis or trans. Expression regulation may work in transcription, translation, splicing, message stability and other levels. Generally, expression regulatory elements for regulating transcription are juxtaposed near the 5'-terminal (i.e., "upstream") of the polynucleotide being transcribed. The expression regulatory element may also be located at the 3'-terminal (i.e., "downstream") of a transcribed sequence or within a transcript (e.g., within an intron). A specific example of the expression regulatory element is a promoter, which is usually located at the 5'-terminal of the transcribed sequence. The expression regulatory element may also comprise a ubiquitous or promiscuous promoter, making it possible to promote the expression of a polynucleotide in many different cell types. The term "tissue-specific expression regulatory element" generally refers to an expression regulatory element that is active in a specific tissue or cell, such as in the liver, brain, central nervous system, spinal cord, eyes, or lungs.

The liver-specific expression regulatory element of the application may comprise a promoter. The promoter may be located at the 5' terminal of the gene of interest and is operably linked to the gene of interest. The promoter may affect the expression of the isolated nucleic acid molecule of the application. For example, the promoter may work in transcription, translation, splicing, message stability and other levels, thereby being able to drive expression of the isolated nucleic acid molecule of the application in many different cell types. In the application, the promoter may be a liver-specific promoter. The liver-specific promoter herein refers to a promoter that is active in the tissues or cells of liver.

In some cases, the liver-specific expression regulatory element of the application may comprise a promoter derived from a dog serpinAl gene or a functional fragment thereof. The promoter derived from the dog serpinAl gene may be a modified variant of a promoter derived from a wild-type dog serpinAl gene. In some cases, the expression regulatory element of the application may comprise a promoter derived from a Xenopus laevis vitellogenin A2 gene or a functional fragment thereof. In some cases, the expression regulatory element may comprise a promoter derived from a Xenopus laevis albumin gene or a functional fragment thereof. The promoter derived from the Xenopus laevis albumin gene may be a modified variant of a promoter derived by a wild-type Xenopus laevis albumin gene.

The liver-specific expression regulatory element of the application may comprise: a) a promoter derived from a dog serpinA1 gene or a functional fragment thereof; and b) a promoter derived from a Xenopus laevis albumin gene or a functional fragment thereof; and

In some cases, the liver-specific expression regulatory element comprises, in order from 5' to 3': the promoter derived from a dog serpinA1 gene or a functional fragment thereof; and the promoter derived from a Xenopus laevis albumin gene or a functional fragment thereof.

In addition, in the application, the isolated nucleic acid molecule may also comprise an intron. The intron may influence the expression of the isolated nucleic acid molecule of the application. For example, the intron may work in transcription, translation, splicing, message stability and other levels, thereby being able to drive expression of the isolated nucleic acid molecule of the application in many different cell types. In some cases, the intron may be located at the 5'-terminal of the isolated nucleic acid molecule of the application. For example, the intron may be located at the 5' terminal of the promoter as defined and is operably linked to the promoter.

In the application, the liver-specific expression regulatory element may comprise a nucleotide sequence as set forth in SEQ ID NO: 1. In the application, the liver-specific expression regulatory element may comprise a nucleic acid sequence having at least 90% (for example, at least 91%, at least 92%, at least 93%, at least 94%, at least 95%, at least 96%, at least 97%, at least 98%, or at least 99%) identity with the nucleotide sequence as set forth in SEQ ID NO: 1.

### Polyadenylation signal sequence

In the application, the isolated nucleic acid molecule may comprise a polyadenylation signal sequence, for example, polyadenylation signal sequences derived from SV40, a bovine growth hormone (BGH) gene, a rabbit beta globulin (RBG) gene, a polyoma virus and a thymidine kinase gene (TK) or variants thereof.

In the application, the polyadenylation signal sequence may be a polyadenylation signal derived from Common vole polyomavirus. In some embodiments, the polyadenylation signal is a polyadenylation signal derived from Common vole polyomavirus isolate KS13.

For example, the polyadenylation signal sequence may comprise a nucleotide sequence as set forth in SEQ ID NO: 3, or comprise a nucleic acid sequence having at least 90% (for example, at least 91%, at least 92%, at least 93%, at least 94%, at least 95%, at least 96%, at least 97%, at least 98%, or at least 99%) identity with the nucleotide sequence as set forth in SEQ ID NO: 3.

The polyadenylation signal sequence (e.g., KS13-polyA signal sequence) may increase the expression level and/or activity of the gene of interest in the vector as defined by at least 20%. For example, the polyadenylation may increase the expression level and/or activity of the gene of interest by at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95% or at least 99%. The expression level here may be determined in the liver or hepatocytes, or may also be determined in the blood.

In some cases, compared with the polyadenylation signal sequences derived from SV40, a BGH gene, an RBG gene, a polyoma virus and a TK gene or variants thereof, the polyadenylation signal sequence (e.g., KS13-polyA signal sequence) may increase the expression level and/or activity of the gene of interest in the vector as defined by at least 20%.

In the application, the isolated polynucleotide may comprise the liver-specific expression regulatory element and the polyadenylation signal sequence. The liver-specific expression regulatory element may comprise the nucleotide sequence as set forth in SEQ ID NO: 1. The polyadenylation signal sequence may comprise the nucleotide sequence as set forth in SEQ ID NO: 3.

### Gene of interest

In the application, the isolated nucleic acid molecule may also comprise a gene of interest, which is operably linked to the liver-specific expression regulatory element. The liver-specific expression regulatory element and the gene of interest may be directly adjacent to each other, or separated by a stretch of inserted nucleotides. In some cases, the liver-specific expression regulatory element and the gene of interest can be separated by an intron, such as the intron of the application. In some cases, no intron is included between the liver-specific expression regulatory element and the gene of interest.

The gene of interest of the application can encode a polypeptide, protein, or polynucleic acid, or may itself be transcribed into a functional or active polynucleic acid, for example, an antisense nucleic acid or an inhibitory oligonucleotide, comprising antisense DNA and RNA (such as miRNA, siRNA and shRNA). In some cases, the gene of interest may encode a protein of interest, which may comprise a reporter protein. The reporter protein may be an enzyme, a fluorescent label, a molecule containing a radioisotope, and a chemiluminescent or electrochemiluminescent molecule. Exemplary reporter proteins comprise, but are not limited to, a green fluorescent protein (GFP), an enhanced green fluorescent protein (eGFP), a yellow fluorescent protein (YFP), an enhanced yellow fluorescent protein (eYFP), a cyan fluorescent protein (CFP), an enhanced cyan fluorescent protein (eCFP), a blue fluorescent protein (BFP), an enhanced blue fluorescent protein (eBFP), MmGFP (Zemicka-Goetz et al., Development, 124: 1133-1137, 1997), dsRed, luciferase and β-galactosidase (lacZ). For example, it may be firefly luciferase and Renilla luciferase.

In some other cases, the gene of interest may be a therapeutic gene. The therapeutic gene can encode a therapeutic peptide, a therapeutic polypeptide, a therapeutic protein, or a therapeutic polynucleic acid. The therapeutic peptide, therapeutic polypeptide, or therapeutic protein may be a peptide, polypeptide, or protein that can be used to restore or replace the function of a defective endogenous peptide, polypeptide, or protein. In some cases, the therapeutic protein or therapeutic polynucleic acid can be used to change the expression level and/or activity of one or more proteins or polynucleic acids in a host cell.

In some other cases, the gene of interest may be a prophylactic gene. The prophylactic gene can encode a prophylactic peptide, a prophylactic polypeptide, a prophylactic protein, or a prophylactic polynucleic acid. The prophylactic peptide, prophylactic polypeptide, or prophylactic protein may be a peptide, polypeptide, or protein that can be used to restore or replace the function of a defective endogenous peptide, polypeptide, or protein. In some cases, the prophylactic protein or prophylactic polynucleic acid can be used to change the expression level and/or activity of one or more proteins or polynucleic acids in a host cell.

In some cases, the protein of interest may participate in or affect cell metabolism, immune response, hematopoietic function, inflammatory response, cell growth and/or proliferation, cell differentiation, and/or stress response. Exemplary proteins of interest may comprise, but are not limited to: factor FVIII, factor FIX, factor FVII, factor FX, interferon-α, interferon-β, interferon-γ, interleukin 1 (IL-1), interleukin 2 (IL-2), interleukin 3 (IL-3), interleukin 4 (IL-4), interleukin 5 (IL-5), interleukin 6 (IL-6), interleukin 7 (IL-7), interleukin 8 (IL-8), interleukin 9 (IL-9), interleukin 10 (IL-10), interleukin 11 (IL-11), interleukin 12 (IL-12), chemokine (CXC motif) ligand 5 (CXCL5), granulocyte colony-stimulating factor (G-CSF), granulocyte-macrophage colony-stimulating factor (GM-CSF), macrophage colony-stimulating factor (M-CSF), stem cell factor (SCF), keratinocyte growth factor (KGF), monocyte chemoattractant protein-1 (MCP-1), tumor necrosis factor (TNF), afamin (AFM), α-galactosidase A, aL-iduronidase, lysosomal α-glucosidase, phenylalanine hydroxylase, lipoprotein lipase, apolipoprotein, low-density lipoprotein receptor (LDL-R), albumin, glucose-6-phosphatase, antibodies, nanobodies, antiviral dominant inactivated proteins, and their fragments, subunits or mutants.

In the application, the gene of interest can encode Factor VIII (FVIII). For example, the gene of interest may encode a B domain-deleted Factor VIII (FVIII) variant. For example, the FVIII variant may comprise an amino acid sequence as set forth in SEQ ID NO: 7, or comprise an amino acid sequence having at least 90% (for example, at least 91%, at least 92%, at least 93%, at least 94%, at least 95%, at least 96%, at least 97%, at least 98%, or at least 99%) identity with the amino acid sequence as set forth in SEQ ID NO: 7.

In the application, the gene of interest may comprise a nucleotide sequence as set forth in SEQ ID NO: 6, or comprise a nucleotide sequence having at least 90% (for example, at least 91%, at least 92%, at least 93%, at least 94%, at least 95%, at least 96%, at least 97%, at least 98%, or at least 99%) identity with the nucleotide sequence as set forth in SEQ ID NO: 6.

In the application, the gene of interest may be a codon-optimized polynucleotide. In the application, the gene of interest may comprise a nucleotide sequence as set forth in SEQ ID NO: 2, or comprise a nucleotide sequence having at least 90% (for example, at least 91%, at least 92%, at least 93%, at least 94%, at least 95%, at least 96%, at least 97%, at least 98%, or at least 99%) identity with the nucleotide sequence as set forth in SEQ ID NO: 2.

In the application, the isolated polynucleotide may comprise, in order from 5' to 3', the liver-specific expression regulatory element, the gene of interest and the polyadenylation signal sequence. The liver-specific expression regulatory element may comprise the nucleotide sequence as set forth in SEQ ID NO: 1. The polyadenylation signal sequence may comprise the nucleotide sequence as set forth in SEQ ID NO: 3.

In the application, the isolated polynucleotide may comprise, in order from 5' to 3', the liver-specific expression regulatory element, the gene of interest and the polyadenylation signal sequence. The liver-specific expression regulatory element may comprise the nucleotide sequence as set forth in SEQ ID NO: 1. The polyadenylation signal sequence may comprise the nucleotide sequence as set forth in SEQ ID NO: 3. The gene of interest may encode the FVIII protein. For example, the gene of interest may comprise a nucleotide sequence as set forth in SEQ ID NO: 2 or 6.

### Other elements

In the application, to facilitate the packaging of a certain component (e.g., the expression regulatory element and/or the gene of interest) of the isolated nucleic acid molecule into a vector (e.g., an AAV vector). For example, a certain component (e.g., the expression regulatory element and/or the gene of interest) of the isolated nucleic acid molecule may be packaged into an AAV1, AAV2, AAV3, AAV4, AAV5, AAV6, AAV7, AAV8, AAV9, AAV10, AAV11, AAV12 or AAV13 vector. For example, a certain component (e.g., the expression regulatory element and/or the gene of interest) of the isolated nucleic acid molecule may be packaged into an AAV8 vector.

The isolated nucleic acid molecule of the application may further comprise AAV inverted terminal repeats (ITRs) located at a 5'-terminal of the enhancer and at a 3'-terminal of the polyadenylation signal sequence. The AAV ITR may be derived from any serotype of AAV, comprising but not limited to, AAV1, AAV2, AAV3, AAV4, AAV5, AAV6, AAV7, AAV8, AAV9, AAV10, AAV11, AAV12 or AAV13, and a naturally occurring or artificial variant thereof. Compared with a wild-type AAV ITR, the nucleic acid sequence comprised in the AAV ITR of the application may be a nucleic acid sequence that has undergone nucleotide insertion, deletion, and/or substitution. Moreover, the AAV ITRs of the isolated nucleic acid molecule may be different or derived from different serotypes, as long as they have the desired function (e.g., the capability of replicating and packaging the gene of interest in gene therapy). In some cases, the AAV ITR of the application may be derived from an AAV serotype selected from the group consisting of: AAV5 and AAV2. For example, the AAV ITR may comprise a nucleotide sequence as set forth in any one of SEQ ID NOs: 4-5.

In a specific case, the nucleotide sequence may be prepared by a cloning technology, or synthetically generated. It may also be prepared by an amplification method. The amplification method comprises polymerase chain reaction (PCR), ligase chain reaction (LCR), transcription-based amplification system (TAS), and self-sustained sequence replication system (3SR). Many methods of cloning, synthesis, and amplification are well known in the art.

In the application, the isolated nucleic acid molecule of the application may comprise, in order from 5' to 3': the liver-specific expression regulatory element, the gene of interest, and the polyadenylation signal sequence. In some cases, the polyadenylation signal sequence may be a KS13-polyA signal sequence. For example, the isolated nucleic acid molecule of the application may comprise, in order from 5' to 3': the liver-specific expression regulatory element, the gene of interest, and the KS13-polyA signal sequence.

For example, the isolated nucleic acid molecule of the application may comprise a nucleotide sequence as set forth in SEQ ID NO: 9.

### Vector, Host Cell and Method

In another aspect, the application further provides a vector, which may comprise the isolated nucleic acid molecule as defined. The vector may be a viral vector, for example, an AAV (e.g., an AAV8), lentivirus, retrovirus, adenovirus, herpes virus, or hepatitis virus vector. The vector may also be a polynucleotide vector, for example, a plasmid, cosmid or transposon.

Applicable vectors have been widely described and are well known in the art. Those skilled in the art would understand that a vector comprising the isolated nucleic acid molecule of the application may also contain other sequences and elements required for the vector to replicate in prokaryotic and/or eukaryotic cells. For example, the vector of the application may comprise a prokaryotic replicon, i.e., a nucleotide sequence capable of guiding the host's own replication and maintenance in prokaryotic host cells (for example, bacterial host cells). The replicon is well known in the art. In some cases, the vector may contain a shuttle element, which enables the vector to adapt to replication and integration in prokaryotes and eukaryotes. In addition, the vector may also comprise a gene capable of expressing a detectable marker (for example, a drug resistance gene). The vector may also have a reporter gene, for example, a gene capable of encoding fluorescent or other detectable proteins.

In some cases, the vector may be a viral vector, for example, an AAV, lentivirus, retrovirus, adenovirus, herpes virus, and hepatitis virus vector. Methods for generating a viral vector containing a nucleic acid molecule (for example, the isolated nucleic acid molecule of the application) as part of a vector genome are well known in the art, and those skilled in the art can perform these methods without excessive experiments. In some other cases, the vector may be a recombinant AAV virion packaging the isolated nucleic acid molecule of the application. A method for producing a recombinant AAV may comprise introducing the isolated nucleic acid molecule of the application into a packaging cell line to induce auxiliary functions of AAV cap and rep genes, and recovering the recombinant AAV from the packaging cell line. Various types of cells can be used as packaging cell lines.

In another aspect, the application further provides a host cell, which may comprise the isolated nucleic acid molecule or vector as defined. In some cases, the host cell can be used to amplify, replicate, package/or purify the nucleic acid molecule or the vector. In some other cases, the host cell can be used to express the gene of interest contained in the isolated nucleic acid molecule or the vector. For example, the isolated nucleic acid molecule or vector of the application can be introduced into a host cell, for example, a liver cell. Those skilled in the art would understand the conditions required for introducing the isolated nucleic acid molecule or the vector into the host cell, as well as the conditions for supporting or promoting the expression of the gene of interest in the cell. In addition, the method may be an in vivo or in vitro method.

The host cell may comprise a prokaryotic cell and a eukaryotic cell. In some cases, the host cell may be a mammalian host cell. For example, when a host cell is used to package the viral vector, the host cell may also be transfected with one or more plasmids, or infected with one or more viruses, which can provide essential accessory molecules for packaging. In some other cases, the host cell may stably express one or more types of accessory molecules from a genome. Those skilled in the art can select an appropriate host cell for the amplification, replication, packaging and/or purification of the vector of the application. In a particular example, in order to allow the isolated nucleic acid molecule or vector as described to express a gene of interest, the host cell may be a cell derived from the liver, for example, a HUH7 cell and a HepG2 cell, or a hepatocyte isolated from a subject.

In another aspect, the application further provides a method for expressing a gene of interest, comprising introducing the isolated nucleic acid molecule as defined or the vector as defined into a host cell, and allowing the gene of interest to be expressed in the host cell.

### Pharmaceutical Composition and Kit

In another aspect, the application further provides a diagnostic or pharmaceutical composition, comprising the isolated nucleic acid molecule, the vector, or the host cell, as defined. AAV vectors and other compositions, medicaments, drugs, biological agents (proteins), for example, pharmaceutically acceptable carriers, excipients, diluents and adjuvants, can be incorporated into the diagnostic and pharmaceutical composition as described. The carriers, excipients, diluents and adjuvants may comprise buffers, antioxidants, proteins, hydrophilic polymers, amino acids, monosaccharides, disaccharides, and other carbohydrates, chelating agents, sugar alcohols, salt-forming counter ions, and/or nonionic surfactants.

In the case where the composition contains a nucleic acid molecule or a nucleic acid molecule vector (e.g., a plasmid), the nucleic acid molecule or nucleic acid molecule vector may be present as "naked DNA" or prepared in a delivery vector, for example, microparticles or nanoparticles, comprising liposomes, micelle, lipid particles, ceramic/inorganic particles, and virus-like particles.

The pharmaceutical composition, method and application described in the application may be administered to a subject in need thereof in a sufficient amount or an effective amount. "Effective amount" or "sufficient amount" refers to an amount provided in a single does or multiple doses alone or in combination with one or more other compositions (therapeutic agents such as drugs), treatments, regimens or therapeutic regimens, and agent combinations to achieve a detectable response of any duration (long-term or short-term), any measurable or detectable extent, or an expected or desired result or benefit of any duration (e.g., lasting for several minutes, hours, days, months, or years, or lasting until a cure). The route of administration is not particularly limited. For example, a therapeutically effective amount of a nucleic acid molecule or vector may be administered to a subject intramuscularly, intravaginally, intravenously, intraperitoneally, subcutaneously, epidermally, intradermally, rectally, intraocularly, pulmonary, intracranially, intraosseously, orally, or nasally. The nucleic acid molecule or vector may be administered in a single dose or multiple doses, and at different intervals.

In another aspect, the application further provides a kit, comprising the isolated nucleic acid molecule as defined, the vector as defined, and/or the host cell as defined. The kit also generally comprises labels or instructions, which comprise the description of components or instructions for using the components therein in vitro, in vivo, or indirectly in vivo.

### Application and Use

In another aspect, the application further provides a method of delivering the isolated nucleic acid molecule as defined into a mammal or a mammalian cell, comprising administering the isolated nucleic acid molecule as defined or the vector as defined to the mammal or the mammalian cell, or contacting the mammal or the mammalian cell with the isolated nucleic acid molecule as defined or the vector as defined.

For example, the method comprises administering the isolated nucleic acid molecule as defined or the vector as defined to the mammal or a secretory cell of the mammal, or contacting the mammal or the secretory cell of the mammal with the isolated nucleic acid molecule as defined or the vector as defined. For another example, the method comprises administering the isolated nucleic acid molecule as defined or the vector as defined to the mammal or an endothelial cell of the mammal, or contacting the mammal or the endothelial cell of the mammal with the isolated nucleic acid molecule as defined or the vector as defined.

The administration may include administration via intravenous, intramuscular, intradermal, subcutaneous, transdermal, mucosal, intratumoral or mucosal routes. The mode of administration may also include transfection, infection or transduction.

The contacting may be direct or indirect. For example, it may be direct injection of cells by microinjection. For another example, the substance can be brought into contact with the cells in vivo by being provided in a culture medium surrounding the cells or administered to a subject.

In another aspect, the application further provides a use of the isolated nucleic acid molecule as defined, the vector as defined, or the host cell as defined in the preparation of a medicament for treating, alleviating, and/or preventing an FVIII-related disease or disorder. The disease or disorder here may comprise hemophilia A, thrombocytopenia, and/or coagulopathy.

In another aspect, the application further provides a method of treating, alleviating and/or preventing an FVIII-related disease or disorder, comprising administering to a subject the isolated nucleic acid molecule as defined, the vector as defined, or the host cell as defined. The disease or disorder here may comprise hemophilia A, thrombocytopenia, and/or coagulopathy.

In another aspect, the application further provides a use of the isolated nucleic acid molecule as defined, the vector as defined, or the host cell as defined for treating, alleviating, and/or preventing an FVIII-related disease or disorder. The disease or disorder here may comprise hemophilia A, thrombocytopenia, and/or coagulopathy.

Not wishing to be bound by any particular theory, the following examples are only to illustrate the nucleic acid molecule of the application that increases the expression level of the gene of interest and application thereof, and are not intended to limit the scope of the invention of the application.

### Examples

### Example 1 Production of recombinant AAV vector

### Nucleotide sequences of elements in a nucleic acid molecule

The isolated nucleic acid molecule of the application comprises, in order from 5' to 3':
the liver-specific expression regulatory element (SEQ ID NO: 1),
the gene of interest (SEQ ID NO: 2),
the KS13-polyA signal sequence (SEQ ID NO: 3), and
AAV ITRs located at the 5' terminal of the liver-specific expression regulatory element and at the 3' terminal of the KS13-polyA, respectively (5'ITR: SEQ ID NO: 4; 3'ITR: SEQ ID NO: 5).

The nucleic acid molecule was named GT001.

Construction of control nucleic acid molecule D1: the liver-specific expression regulatory element in GT001 was replaced with a promoter having a nucleotide sequence as set forth in SEQ ID NO. 10.

Construction of control nucleic acid molecule D2: the KS13-polyA signal sequence in GT001 was replaced with nucleic acid is a SPA poly A, SV40 polyA or BGH polyA.

### Packaging of AAV8 vector

HEK293 cells were inoculated at a concentration of 4×10⁶/100 ml in diameter onto a plate of a DMEM medium containing 10% FBS, and cultured overnight at 37 °C in a humid environment bearing 5% CO₂. In the next day, a PEI transfection mixture containing the nucleic acid molecule of the application or the control nucleic acid molecule, an AAV8 capsid protein and a helper plasmid was prepared, then added to a medium, and 6 h after transfection, the medium was replaced with a fresh DMEM medium containing 10% FBS, and 72 h after transfection, cells were harvested. The cells were resuspended in a buffer (pH=8) containing 100 mM NaCl, 2 mM MgCl₂ and 10 mM Tris, and stored at -80 °C.

### Purification and quantitative analysis of AAV8 vector

HEK-293 cells containing rAAV8 underwent three freeze-thaw cycles, and then were treated with 50 U/mL Benzonase for 30 min at 37 °C to remove unencapsulated DNA, and then centrifuged at 3,000 g for 10 min to be pelleted. The supernatant was transferred for ultracentrifugation. An iodixanol centrifugal system was prepared. The iodixanol solution was added at a gradient of four concentrations 17%, 25%, 40%, and 60% in order to a 33ml Optiseal tube (Beckman) by using a 10ml syringe. The iodixanol solution of each concentration was added slowly into the Optiseal tube from the bottom: 6 ml 17% solution, 6 ml 25% solution, 5 ml 40% solution, and 4 ml 60% solution. Then, a sample name was marked on the top of the Optiseal tube and a line mark was made at a junction between 40% and 60%. Then, the supernatant was carefully added to a centrifuge tube using a test tube and centrifuged at 53,000 g for 2 h and 40 min at 14 °C.

The needle of a 5ml syringe was then inserted into the Optiseal tube along the line mark (the junction line between 40% and 60%) made before to take the 40% portion of the solution (about 2-3 ml) into another 15 ml tube. The virus solution was added to an equilibrated 100K Centrifuge filter and supplemented with 1× PBS (10⁻⁴ F188) to approximately the 50 ml line, and then centrifuged at 3,500 rpm for 10 min. The waste liquid was removed and the virus solution was supplemented with 1× PBS (10⁻⁴ F188) to the top and then centrifuged at 3,500 rpm for 10 min. Such operations were repeated three times. 300-500 ul of 1× PBS (10⁻⁴ F188) was added to harvest the virus. The virus was transferred to a 1.5 ml EP tube. Then, the purified AW vector genome was quantitatively analyzed by qPCR with a kit according to instructions, and the titer of a virus stock solution was determined.

### Example 2 The repair ability of the isolated nucleic acid molecule of the application on a hemophilia A mouse model

The recombinant AAV8 vector obtained in Example 1 was injected into tail vein of hemophilia A disease model mice at a dose of 4×10¹¹ vg/kg. The FVIII activity in the serum was determined 2 weeks after the injection to detect the symptoms of the mice. According to the results shown in FIG. 1, the isolated nucleic acid molecule GT001 of the application can restore FVIII expression in the hemophilia A mice.

### Example 3 Detection of expression activity of the isolated nucleic acid molecule of the application

Similarly, the control nucleic acid molecules D1 and D2 obtained in Example 1 and the isolated nucleic acid molecule GT001 of the application were packaged with AAV8, and the recombinant AAV8 vector was injected into the tail vein of the hemophilia A disease model mice for restoring the FVIII expression. The results show that compared with the control nucleic acid molecules D1 and D2, the isolated nucleic acid molecule GT001 of the application increases the expression activity of the FVIII variant.

FIG. 2 shows the expression activity of the gene of interest FVIII over different regulatory elements. For example, D1 constructed by replacing the promoter of the application with a control promoter has a significantly lower expression effect than the specific expression vector of the application. For another example, D2 obtained by replacing the polyA signal sequence of the application with a control poly A, such as SPA polyA, has a significantly lower expression effect than the specific expression vector of the application. The results show that the specific isolated nucleic acid molecule GT001 of the application can restore the expression of FVIII.

### Example 4 Detection of expression activity of the isolated nucleic acid molecule of the application

By optionally replacing the FVIII gene in the expression nucleic acid molecule of the application with other genes of interest, such as a FIX gene, the expression nucleic acid molecule of the application can exhibit a similar effect of increasing expression activity.

FIG. 3 shows the expression activity of the expression nucleic acid molecule of the application for optionally expressing a gene of interest. Various molecules were packaged with AAV8, and the recombinant AAV8 vector was injected into the tail vein of hemophilia B disease model mice at a dose of 6×10¹¹ vg/kg. The FIX activity in the serum was determined 2 weeks after injection. The results show that the GT001-F9 molecule obtained by replacing the gene of interest in the specific isolated nucleic acid molecule GT001 of the application with the FIX gene can restore the FIX expression in hemophilia B mice. The D1-F9 molecule obtained by replacing the gene of interest in the control molecule D1 (comprising the control promoter) with the FIX gene has a significantly lower expression effect than the specific expression vector of the application.

The foregoing detailed description is provided by way of explanation and examples, and is not intended to limit the scope of the appended Claims. Various changes of the embodiments listed in the application until now would be obvious to those of ordinary skills in the art, and should be kept within the scope of the appended Claims and equivalents thereof.

## Claims

1. An isolated nucleic acid molecule, comprising, from 5' to 3', a liver-specific expression regulatory element and a gene of interest operably linked to said liver-specific expression regulatory element, wherein compared with an expression regulatory element with a nucleotide sequence as set forth in SEQ ID NO: 10, said liver-specific expression regulatory element increases the expression level and/or activity of said gene of interest by at least 50%.

2. The isolated nucleic acid molecule according to Claim 1, wherein said liver-specific expression regulatory element comprises:
a) a promoter derived from a dog serpinA1 gene or a functional fragment thereof; and
b) a promoter derived from a Xenopus laevis albumin gene or a functional fragment thereof.

3. The isolated nucleic acid molecule according to any one of Claims 1-2, wherein said liver-specific expression regulatory element comprises a nucleotide sequence as set forth in SEQ ID NO: 1.

4. The isolated nucleic acid molecule according to any one of Claims 1-3, comprising a polyadenylation signal sequence located at a 3 '-terminal of said gene of interest.

5. The isolated nucleic acid molecule according to Claim 4, wherein said polyadenylation signal is a polyadenylation signal derived from *Common vole polyomavirus.*

6. The isolated nucleic acid molecule according to any one of Claims 4-5, wherein said polyadenylation signal is a polyadenylation signal derived from *Common vole polyomavirus* isolate KS 13.

7. The isolated nucleic acid molecule according to any one of Claims 4-6, wherein said polyadenylation signal comprises a nucleotide sequence as set forth in SEQ ID NO: 3.

8. The isolated nucleic acid molecule according to any one of Claims 1-7, wherein said gene of interest encodes a protein of interest, and said protein of interest comprises a reporter protein, a therapeutic protein and/or a prophylactic protein.

9. The isolated nucleic acid molecule according to any one of Claims 1-8, wherein said gene of interest encodes a B domain-deleted Factor VIII (FVIII) variant.

10. The isolated nucleic acid molecule according to Claim 9, wherein said FVIII variant comprises an amino acid sequence as set forth in SEQ ID NO: 7.

11. The isolated nucleic acid molecule according to any one of Claims 1-10, wherein said gene of interest comprises a nucleotide sequence as set forth in SEQ ID NO: 2 or 6.

12. The isolated nucleic acid molecule according to any one of Claims 1-11, comprising, in order from 5' to 3': said liver-specific expression regulatory element, said gene of interest, and said polyadenylation signal sequence.

13. The isolated nucleic acid molecule according to any one of Claims 1-12, further comprising AAV inverted terminal repeats ITRs located at a 5'-terminal of said liver-specific expression regulatory element and at a 3'-terminal of said polyadenylation signal sequence.

14. The isolated nucleic acid molecule according to Claim 13, wherein said AAV ITR is derived from an AAV serotype selected from the group consisting of: AAV5 and AAV2.

15. The isolated nucleic acid molecule according to any one of Claims 13-14, wherein said AAV ITR comprises a nucleotide sequence as set forth in any one of SEQ ID NOs: 4-5.

16. The isolated nucleic acid molecule according to any one of Claims 1-15, comprising a nucleotide sequence as set forth in SEQ ID NO: 9.

17. A vector, comprising the isolated nucleic acid molecule according to any one of Claims 1-16.

18. The vector according to Claim 17, which is a viral vector or a polynucleotide vector.

19. The vector according to any one of Claims 17-18, which is a plasmid, a cosmid or a transposon.

20. The vector according to any one of Claims 17-19, which is a viral vector, said viral vector comprising an AAV vector.

21. The vector according to Claim 20, wherein said AAV vector is an AAV8 vector.

22. A host cell, comprising the isolated nucleic acid molecule according to any one of Claims 1-16 and/or the vector according to any one of Claims 17-21.

23. A diagnostic or pharmaceutical composition, comprising the isolated nucleic acid molecule according to any one of Claims 1-16, the vector according to any one of Claims 17-21, and/or the host cell according to Claim 22.

24. A method of expressing a gene of interest, comprising introducing the isolated nucleic acid molecule according to any one of Claims 1-16 or the vector according to any one of Claims 17-21 into a host cell, and allowing said gene of interest to be expressed in said host cell.

25. A kit, comprising the isolated nucleic acid molecule according to any one of Claims 1-16, the vector according to any one of Claims 17-21, and/or the host cell according to Claim 22.

26. A method of delivering the isolated nucleic acid molecule according to any one of Claims 1-16 into a mammal or a mammalian cell, comprising administering the isolated nucleic acid molecule according to any one of Claims 1-16 or the vector according to any one of Claims 17-21 to said mammal or mammalian cell, or contacting said mammal or mammalian cell with the isolated nucleic acid molecule according to any one of Claims 1-16 or the vector according to any one of Claims 17-21.

27. A use of the isolated nucleic acid molecule according to any one of Claims 1-16, the vector according to any one of Claims 17-21, and/or the host cell according to Claim 22 in the preparation of a medicament for treating, alleviating, and/or preventing an FVIII-related disease or disorder.

28. The use according to Claim 27, wherein said disease or disorder comprises hemophilia A, thrombocytopenia, and/or coagulopathy.
